(19)

Europäisches
Patentamt
European
Patent Office
Office européen
des brevets

(11) **EP 4 502 588 A1**

(12) **EUROPEAN PATENT APPLICATION**

(43) Date of publication:
**05.02.2025  Bulletin 2025/06**

(21) Application number: **23189781.0**

(22) Date of filing: **04.08.2023**

(51) International Patent Classification (IPC):
**G01N 27/327** (2006.01)    **G01N 33/543** (2006.01)

(52) Cooperative Patent Classification (CPC):
**G01N 27/3277; G01N 33/5438**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **UAB "ARS Lab"**
**08437 Vilnius (LT)**

(72) Inventors:
• **Ramanavicius, Arunas**
**Vilnius (LT)**

• **Kvaraciejus, Gediminas**
**Vilnius (LT)**
• **Zukauskas, Sarunas**
**Vilnius (LT)**
• **Ramanavicius, Simonas**
**Vilnius (LT)**

(74) Representative: **Zaboliene, Reda**
**Metida**
**Business center Vertas**
**Gyneju str. 16**
**01109 Vilnius (LT)**

(54) **METHOD FOR THE DETERMINATION OF ELECTROCHEMICAL SIGNAL BASED ON THE ASSESSEMENT OF CYCLIC VOLTAMMETRY RESULTS**

(57)    A method of electrochemical signal determination based on assessment of cyclic voltammetry results using second derivative is designed and disclosed. The method comprises obtaining cyclic voltammetry data utilizing an electrochemical cell using a two-, three-, or multi- electrode setup. The obtained data is then algorithmically assessed by first applying second order derivative. Then by the reduction of system noise by utilizing the Savitzky-Golay mathematical filter, we further assess the data by extracting the system baseline, signal position using the second derivative peak position, and by extracting the signal amplitude. By utilizing this electrochemical data processing algorithm, we obtain more repeatable results from cyclic voltammograms, which not have distinct oxidation and/or reduction peaks.

Fig. 5

EP 4 502 588 A1

**Description**

TECHNICAL FIELD

The invention relates to new assessment of electrochemical cyclic voltammetry data obtained utilizing a low-volume measurement system and a potentiostat to allow for accurate measurements when clear, strong oxidation and/or reduction peaks are not observed, and it is difficult to reproducibly locate the exact peak positions in the cyclic voltammogram.

BACKGROUND ART

Various electrochemical cells and systems are developed depending on demands and application areas. For sensing and biosensing purposes mostly miniature electrochemical cells are required, however, recently applied procedures, which are necessary for analysis, are at least partly manual. The reduction of electrode dimensions is also very important since it enables to reduce the background noise of the sensor and by this to increase the sensitivity and/or selectivity of the sensor.

We have developed a novel miniature electrochemical cell equipped with a potentiostat and a low-volume measurement system. This advanced solution addresses the shortcomings of previous methods, which often involve labour-intensive manual procedures.

Common techniques and methods recently used for the assessment of electrochemical signals have numerous disadvantages, especially if cyclic voltammetry is applied, such as: very long-lasting and tedious procedures and mostly requires many analysis steps and calculations, which sometimes are performed manually. Depending on the number of analytical signals they can overlap, all of which make the analysis difficult to interpret. Moreover, traditional assessment of a cyclic voltammetry-based signal is not always suitable. Therefore, the development of new analytical signal assessment methods is demanded.

Some analytical cyclic voltammetry signal assessment techniques can be automated, US patent application No. US9841403B2 discloses a method to assess analyte concentration by combining pulse voltammetry with cyclic voltammetry and measuring system adsorption ability, Chinese patent CN102141538B details a method by calculating a working curve based on the determination of the quantitative proportional relationship between oxide peak currents, Chinese patent CN109444245B by suggesting a calibration curve system utilized in electroplating.

However, the determination of electrochemical signals antibody/antigen is very different in comparison to that of operation of above described. Therefore, the determination of electrochemical signal in such a case is much more complex, and for this reason the determination of analytical signal is much more sophisticated in comparison to the determination of analytical signal described in above mentioned patents.

The present invention is dedicated to overcoming the above mentioned shortcomings and for the achievement of further additional advantages over prior art and by possibility to apply in electrochemical systems dedicated for cyclic voltammetry based determination of analytes, such as protein (antigen, antibody, other specifically interacting proteins, etc.), organic compounds (heme, PQQ, etc,) ions (heavy metal ions, organic ions, etc.) concentrations in biological fluids and in other water-based solutions.

BRIEF DESCRIPTION OF THE INVENTION

The invention presents a novel miniature electrochemical cell integrated with a potentiostat and a low-volume measurement system, tailored to streamline sensing and biosensing applications. Once the data is obtained, it uses an algorithmic method for the registration of electrochemical signal with two-, three-, multi-electrode systems comprising at least one electrode modified by materials, which advances selectivity and or sensitivity towards the determined analyte.

The method includes using an electrochemical approach that utilises the second derivative for the determination of an analytical signal.

(1) Assemble the prepared electrode into the manufactured electrochemical system.

(2) Preparation and electrochemical registration of cyclic voltammogram in the presence of aliquot. This includes adding the aliquot to the measurement chamber.

(3) Conduct the electrochemical measurement.

(4) If necessary (when signal noise exceeds 50 dB), Mathematical smoothing of the unprocessed data using the Savitzky-Golay mathematical smoothing algorithm (filtering) algorithm is performed.

(5) Second order derivative of the processed cyclic voltammetry data is calculated.

(6) The determination of stabile measurement baseline.

(7) The determination of the signal position, where the variation of second order derivative is the most significant.

(8) Determination of the signal value.

The invention encompasses the application of here developed electrochemical signal assessment method to any number of analytes utilizing the electrochemical system.

BRIEF DESCRIPTION OF THE DRAWINGS

Features of the invention believed to be novel and inventive are set forth with particularity in the appended claims. The invention itself, however, may be best understood by reference to the following detailed description of the invention, which describes exemplary embodiments, given in non-restrictive examples, of the invention, taken in conjunction with the accompanying drawings.

Fig. 1. The image depicts a typical configuration of devices utilized in the electrochemical measurement method.
Fig. 2 shows the principal scheme of electrochemical system in the measurement chamber used for the determination of interaction between immobilized antigen (rS-protein of SARS-CoV-2 virus) with specific antibody against the rS protein.
Fig. 3 illustrates an exemplary cyclic voltammogram, demonstrating the application of the present invention to analyze the redox behavior of an indistinct peak corresponding to the analyte.
Fig. 4 shows the second order derivate of the anodic current of the cyclic voltammogram presented in Fig. 3.
Fig. 5 shows a mathematical interpretation of the analytical signal, (a) derivative minimum, (b) linear dependence of the calculated second derivative on the electrode potential, (c) baseline of the signal, (d) recorded analytical signal.
Fig. 6 shows immunoassay system grading curve (analytical signal vs. antibody concentration in the samples). a) squares - signal calculated by evaluating the 'linear component'. b) circles - signal calculated by evaluating the peaks of the cyclic voltammograms.
Fig. 7 shows a regression analysis of the grading curves ('calibration plots') in semi-logarithmic coordinates (a) determined by evaluating the values of $\Delta J_i$, which are calculated using the analytical signal evaluation methodology presented in Fig. 3.6. 5, and (b) evaluating the peak currents of the cyclical voltammogram peaks only.

Preferred embodiments of the invention will be described herein below with reference to the drawings. Each figure contains the same numbering for the same or equivalent elements.

DETAILED DESCRIPTION OF THE INVENTION

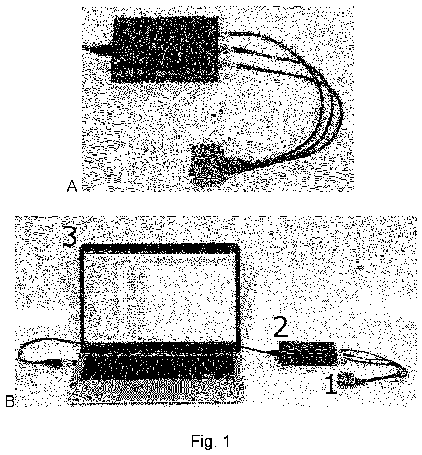

It should be understood that numerous specific details are presented to provide a complete and comprehensible description of the invention embodiment. However, the person skilled in the art will understand that the embodiment examples do not limit the application of the invention, which can be implemented without these specific instructions. Well-known methods, procedures and components have not been described in detail for the embodiment to avoid misleading. Furthermore, this description should not be constraining the invention to given embodiment examples but only as one of the possible implementations of the invention.
A system comprises various components: a miniature electrochemical cell, into which a prepared electrode is integrated. The cell's compact design helps to minimize background noise. The setup also includes a measurement chamber (1) with a capacity of 200 microliters. The entire electrochemical system is connected to a potentiostat (2) - an essential device for controlling and measuring the current within the cell. Additionally, a computer (3) is included in this configuration to record and analyse the electrochemical signals (Fig. 1).
The current invention pertains to an advanced system for electrochemical measurements, incorporating a miniature electrochemical cell, an integrated potentiostat (2), a low-volume measurement system, and a unique algorithmic evaluation technique of the electrochemical system. The invention offers a robust solution for achieving sensitive and selective electrochemical measurements, even when clear oxidation and/or reduction peaks are not observed in the cyclic voltammogram, or when multiple oxidation and/or reduction peaks are overlapping.
The miniature electrochemical cell serves as the centerpiece of the system. The cell is designed to accommodate a small electrode. The low overall volume allows for the reduction in distance between the electrodes, which further reduces the electrical resistance of the system. The combination of these effects is crucial for the reduction of background noise. The lower noise leads to increased sensitivity and selectivity of the sensor, improving the overall quality of the electrochemical measurements. The reduction in volume further reduces the amount of reactants needed for the electrochemical measurement, reducing the cost of the analysis.

The electrode is prepared following standard electrochemical preparation techniques. The prepared electrode is then assembled into the miniature electrochemical cell, ensuring a secure and stable platform for the electrochemical measurements.

The electrochemical cell is connected with a potentiostat (2). This device controls the potential applied to the cell and measures the current in the electrochemical cell, registering the electrochemical signal. The potentiostat (2) is a critical component for maintaining the accuracy of the measurements.

One of the unique features of the setup is the low-volume measurement system. This system allows for testing with minimal sample amounts, effectively reducing costs and increasing the feasibility of large-scale testing. The low-volume measurement system is enabled by an internal measurement chamber within the electrochemical cell. The chamber (1) is designed to hold a maximum volume of 200 microliters, which is a considerably small volume compared to traditional electrochemical cells. Despite its small size, the chamber maintains a high degree of precision and accuracy in the measurements.

In the next step, the aliquot is added into the internal measurement chamber (1). The small volume of the chamber (1) ensures that only a minimal amount of the sample is required for the electrochemical measurement, thereby maximizing the efficiency of the system.

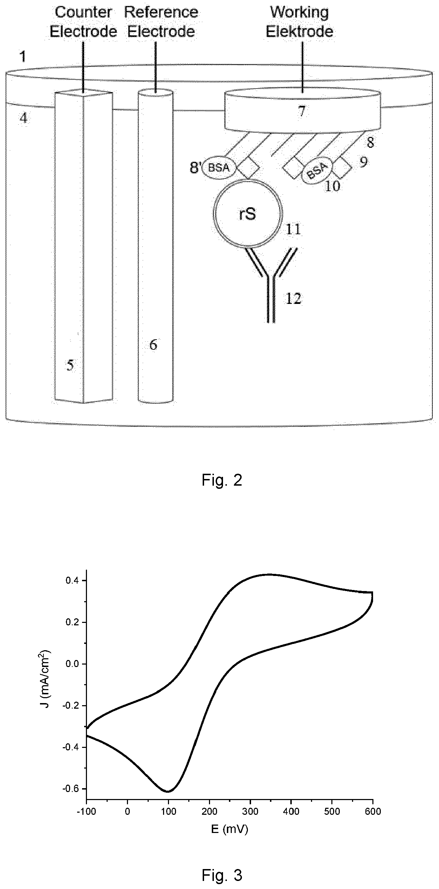

Fig. 2 The figure illustrates the principal scheme of an Electrochemical system deployed within a specialized measurement chamber. This chamber is explicitly designed for the determination of the interaction between an immobilized antigen and a specific antibody targeting the rS-protein of the SARS-CoV-2 virus. The depiction includes various essential components, each of which is numerically labelled to provide a comprehensive understanding of the system's structure and function:

1. Low Volume Reaction Chamber: This section encapsulates the site of the chemical reactions, allowing for precise control and observation. It ensures a controlled environment for the reaction.

4. Buffered Electrolyte Solution: This solution facilitates the transfer of electric charge within the system, maintaining stable conditions for accurate measurements.

5. Platinum Counter Electrode: Acting as an auxiliary electrode, it helps in balancing the charge during electrochemical reactions.

6. Saturated Silver Silver Chloride Reference Electrode: This component serves as a stable reference point for the voltage in the system, allowing for consistent and accurate measurements.

7. Gold Substrate: Utilized as the working electrode.

8. SAM (Self-Assembled Monolayer) Layer: This molecular layer is carefully arranged on the gold substrate, providing the foundation for the immobilization of the antigen.

9. Amide Group Formed Through EDC-NHS Coupling Reaction: This bond assists in attaching the SARS-CoV-2 spike protein (rS-protein) to the SAM layer.

10. Open SAM Blocked by BSA (Bovine Serum Albumin): These sections refer to the areas where the SAM layer is not bonded to the rS-protein, preventing nonspecific binding.

11. SARS-CoV-2 Spike Protein: This is the specific antigen that is immobilized in the system, allowing for the targeted study of its interaction with antibodies.

12. Anti-SARS-CoV-2 Spike Antibody: This component represents the specific antibody that targets the SARS-CoV-2 spike protein, forming the basis for the interaction study within the chamber.

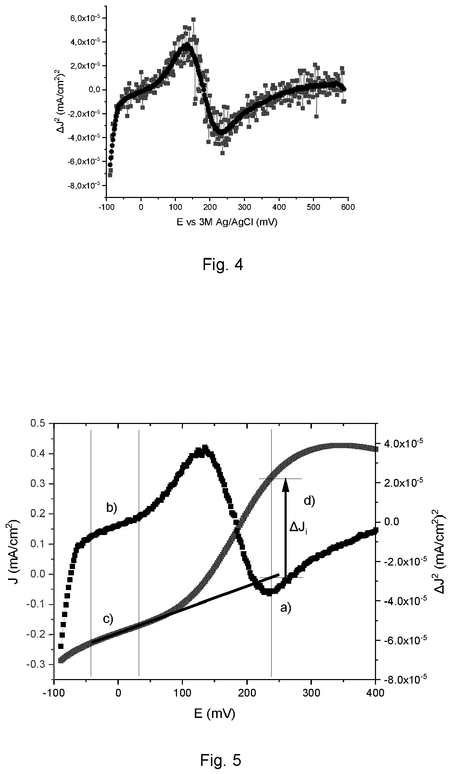

Parts (4, 5, 6) are electrically connected using a conductive aqueous media with the tested analyte (8). And analysing data based on the descriptions provided in Fig. 4 and Fig. 5. In all embodiments of the invention electrodes are conducting electrodes.

Electrochemical system used for the determination of interaction between immobilized antigen (rS-protein of SARS-CoV-2 virus) with specific antibody against the rS protein is provided in Fig. 2 in which a reaction chamber (1) provides a stable and consistent environment for the electrochemical reactions to occur. This test environment is designed to contain all the other components and facilitate efficient electron transfer. A physiological buffer reaction electrolyte (4) is employed to ensure that the pH of the reaction environment is maintained. This buffer acts as an intermediary in electron transfer and minimizes any changes in the pH, allowing the reactions to take place in a controlled and stable setting. A platinum counter electrode (5) serves as the secondary electrode in the electrochemical system. This electrode is designed to balance the current in the electrochemical cell by participating in an oxidation or reduction reaction opposite to the one occurring at the working electrode. A silver/silver chloride reference electrode (6) is used to maintain a stable and known potential against which the potential of the working electrode can be measured. This ensures accurate and reliable electrochemical readings during the experiment. A gold substrate (7) is connected as the working electrode in the electrochemical system. A Self-Assembled Monolayer (SAM) (8) composed of a mix of mercaptoundecanoic acid and 6-mercapto-1-hexanol is layered on the gold substrate. These layers serve as a scaffold, enabling controlled attachment of other substances onto the electrode. An amide group (9) formed through the EDC-NHS reaction is used to attach proteins to the SAM layer. This method allows for the creation of a covalent bond between carboxyl groups on the SAM and the amine groups on the protein, ensuring a stable and durable attachment. Bovine albumin serum albumin (BSA) (10) is attached to the previously formed amide groups. This protein serves as a control or blocking agent, minimizing nonspecific interactions on the electrode surface. Bovine albumin serum albumin (BSA) (8') is non-specifically adsorbed to SAM layer and protects immunosensing system from nonspecific interaction. Recombinant S (rS) spike protein (11) is attached through the same amide group. This protein, derived from viruses, is the primary target of this electrochemical cell and forms the basis of the specific biological interaction that is being studied. Anti-rS spike antibody (12) is attached to the rS spike protein. This attachment forms the specific bio-recognition element in the system, creating a highly specific and sensitive response to the presence of the rS spike protein. Parts (5, 6, 7) are electrically connected using the physiological buffer reaction electrolyte (4), facilitating the transfer of electrons and ensuring the operation of the electrochemical cell. The interactions of the various layers, specifically the SAM layer (8), the proteins (10, 11), and the antibody (12) form the basis for the detection and quantification of the specific analyte under investigation. The results from this setup are analysed based on the descriptions provided in the accompanying figures. In all embodiments of the setup, the electrodes used are conducting electrodes.

A prepared SARS-CoV-2 protein-modified electrode was subjected to an electrochemical immunoassay embodiment example for the determination of antibody concentrations in solutions of different concentrations prepared using serum solutions from a SARS-CoV-2 treated patient. It should be noted that the anti-SARS-CoV-2 antibody-containing serum was obtained from the blood serum of a volunteer who was immunised with the Vaxzevria vaccine against Covid-19 for the first time and who developed the disease 2 weeks later (blood collection was performed at 1 month after the symptoms of Covid-19 vanished).

A sample of solutions containing different concentrations of anti-SARS-CoV-2 antibodies (anti-rS) was dropped onto the surface of the SARS-CoV-2 protein-modified electrode and incubated for 30 minutes. Anti-rS antibody solutions at concentrations ranging from 1 to 100 nM were used to generate a gradation curve ('calibration plot'). The assay was performed by successive incubations of the electrodes in increasing concentrations of serum antibody solutions, and after each incubation step, cyclic voltammetry was performed by varying the electrode potential between -100 mV and 600 mV against Ag/AgCl at a rate of 50 mV/s. Two cyclic voltammograms were recorded and the position of the anodic peak at 200 mV of the second cyclic voltammogram and the amperometric signal current at the peak were analysed in order to assess the analytical signal (Fig. 5).

The data for cyclic voltametric cycle was obtained by electrochemical assessment of SARS-CoV-2 virus protein-modified gold electrode after it was incubated with 11 nM of antibodies against SARS-CoV-2 virus spike protein (anti-RS antibody) solution. The working electrode is an Au electrode with a surface area of 0.071 cm$^2$; reference electrode is a commercial saturated silver/silver chloride electrode; counter electrode is platinum with a working area of around 1 cm$^2$. Experiment was performed in 0.1 M Phosphate saline buffer with 2 mM each of potassium hexacyanoferrate(iii) and potassium hexacyanoferrate(ii). Using the cyclic voltammogram data obtained from the analysis of electrochemical data after the incubation of electrodes in samples with 11 nM anti-rS antibody concentrations, additionally shown in Fig. 2, we recorded the position of the oxidation peak, which is not very well defined in the graph under study, and therefore makes it difficult to assess the analytical signal with high accuracy. Therefore, the second order derivative of the anodic voltammogram was fitted to the potential dependence of the analytical signal. The cyclic voltammogram data has been processed using the Savitzky-Golay mathematical filtering method using a 25-point interval. After filtering the data, we obtain a new set of noised current-voltage data with significantly reduced noise level, which is displayed as the inner line (Fig. 3). The second derivative was calculated by separating the anodic part of the cyclic voltammogram and differentiating it:

$$f''(x) = \frac{d^2 y}{dx^2} \quad \text{(Formula 1)}$$

The transformation is quite sensitive to the 'noise' of the analytical signal and makes it even more pronounced, since the recorded 'noise' during the recording of the cyclic voltammogram has a relatively large influence on the second order derivative of the recorded signal. Since we cannot completely avoid the 'noise' of the analytical signal recording and/or the 'noise' of the electronic equipment, we have therefore smoothed the data using an appropriate mathematical smoothing algorithm. The results of the second derivative calculation were smoothed using the Savitzky-Golay mathematical smoothing algorithm (Formula 1). A 25-point stretch was used for the smoothing. Fig. 3 shows the data recorded before and Fig. 4 shows the data after the mathematical smoothing - inner line.

The mathematical model of interpretation of the analytical signal was provided by (a) derivative minimum, (b) linear dependence of the calculated second derivative on the electrode potential, (c) baseline of the signal, (d) recorded analytical signal. The difference ($\Delta$Ji) between the extrapolated straight line plotted over the section of the cyclic voltammogram in which the second derivative of the second derivative is linearly related to the electrode potential and the currents are recorded at the value of the potential at which the second derivative of the signal function of the cyclic voltammogram reaches a minimum value. Using the second derivative of the cyclic voltammogram function, we have determined the baseline of the system and the 'linear component' of the potential dependence of the second derivative. The position of the first minimum of the second derivative, found after the first maximum (Fig. 5a), coincides with the end of the linear component of the anodic peak, with the points on the line before the peak is formed (Fig. 5b). We have found a zone where the linear dependence of the points on the potential is characterised by a correlation coefficient r2 > 0.99, and by evaluating this zone of the analytical signals, we determine the baseline function of the CV (Fig. 5c). The analytical signal to be evaluated is equivalent to the difference between the recorded current density at the second derivative minimum and the interpolated baseline current density (Fig. 5d).

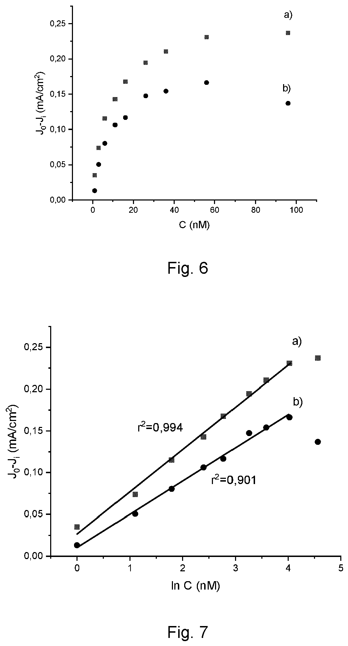

Using the methodology described in the previous paragraph, we generated a gradient curve ('calibration plot') of the immune sensor showing the dependence of the calculated analytical signal on the concentration of the antibody in the sample (Fig. 6). This assay was carried out according to the protocol below. The sample was applied to the surface of the electrode and incubated for 30 min with different concentrations of anti-SARS-CoV-2 antibody (anti-rS) solutions. Solutions with anti-RS antibody concentrations ranging from 1 to 100 nM were used to generate a gradation curve ('calibration plot'). The assay was performed by incubating the electrodes successively in increasing concentrations of serum solutions containing antibodies, and after each incubation step, cyclic voltammetry was performed by varying the electrode potential between -100 mV and 600 mV against Ag/AgCl at a rate of 50 mV/s. Two cyclic voltammograms were recorded and the position of the anodic peak at 200 mV of the second cyclic voltammogram and the amperometric signal current at the peak were analysed to evaluate the analytical signal. The modified electrode was then regenerated and incubated in another solution containing an appropriate antibody concentration. As the immobilised antibody concentration increased, a decrease in the peak of the recorded peak current and a shift of this peak to more positive potential values was observed. The dependence of the analytical signals on the concentration of the antibody in the sample is reproduced in Figure 7.

The amplitude of the analytical signals shown in the grading curve (Fig. 6a) is higher for the $\Delta$Ji values calculated using the analytical signal evaluation methodology in Fig. 5, compared to the signals calculated using only the peak current values of the cyclic voltammograms (Fig. 6b). Therefore, the methodology used allows a more accurate determination of the antibody concentrations in the samples tested. By analysing the test data in semi-logarithmic coordinates with respect to concentration and by regression analysis of both data sets, we found that a linear regression is observed in such semi-logarithmic coordinates (Fig. 7), with satisfactory correlation coefficients squared values. It should be noted that when evaluating the analytical signals presented in the grading curve ('calibration plot') (Fig. 6a), the $\Delta$Ji calculated using Fig. 5. The correlation coefficients squared values of the analytical signal estimation methodology presented in Fig. 5 are significantly higher ($r^2$ =0.994) compared to the correlation coefficients squared value ($r^2$ =0.900) calculated by estimating the dependence of the peak currents of the cyclic voltammograms only on the antibody concentrations in the samples analysed. It should be noted that the analytical signal value calculated for the maximum antibody concentration measured in these studies does not correlate with the above linear relationships using both methods of analytical signal estimation and, therefore, these values are not considered during the analyses, and when analysing solutions with higher concentrations of antibodies, it is advisable to dilute the solutions prior to the analysis.

By evaluating the data from the grading/calibration curves (Fig. 7), we determined the limit of detection (LOD) and limit of quantification (LOQ) for the immunoassay systems. The three lowest concentrations were used to determine these characteristics and the LOD and LOQ are shown in Table 1.

Table 1. Standard error, angle of inclination of a straight line, limit of detection (LOD) and limit of quantification (LOQ)

|  | By linear component | By maximum |
| --- | --- | --- |
| Standard error of the y-axis data | 0,005516, nM | 0,00844, nM |
| Angle of inclination of a straight line | 0,015851 | 0,013188 |

(continued)

|  | By linear component | By maximum |
|---|---|---|
| Limit of detection (LOD) | 1,148267, nM | 2,11199, nM |
| Limit of quantification (LOQ) | 3,479598, nM | 6,399969, nM |

The uncertainties resolved in this activity were: the selection of optimal electrochemical system parameters, selection/improvement of analytical signal estimation models for the anti-rS antibody detection system. The technological challenges related to the selection of electrochemical cyclic voltammetry modes are solved. The cyclic voltammetry characteristics that can be evaluated as an analytical signal is selected and a relatively simple model for the evaluation of the analytical signals of cyclic voltammograms is applied, which are suitable for the anti-SARS-CoV-2 antibody detection system. A methodology has been developed to evaluate the cyclic voltammogram signals in such a way that they reflect the interaction of immobilized SARS-CoV-2 virus rS proteins with specific antibodies against these proteins, which are present in aliquote. It has been demonstrated that the analytical signal estimation model can be improved, if necessary, by a more sophisticated mathematical treatment of the analytical signal, by applying a second derivative function to the analytical signal, and by evaluating the differences between the direct signal and the second derivative functions in the linear parts of these functions. The performance of the mathematical model for the recording of this analytical signal has been evaluated and compared with the performance of a simple/standard method of analysing the analytical signal by simply estimating the oxidation peak current.

Although numerous characteristics and advantages together with structural details and features have been listed in the present description of the invention, the description is provided as an example fulfilment of the invention. Without departing from the principles of the invention, there may be changes in the details, especially in the form, size, and layout, in accordance with the most widely understood meanings of the concepts and definitions used in claims.

## Claims

1. An electrochemical system configured to provide electrochemical signal, wherein the system comprises:

   (a) dielectric material containment cell chamber for the test environment;

      a) gold electrode configured as working electrode in the electrochemical system;
      b) platinum electrode configured to work as electrode in the electrochemical system;
      c) tested analyte;

   wherein gold electrode a) and platinum electrode b) are electrically connected using a conductive aqueous media with the tested analyte c).

2. The electrochemical system according to claim 1, wherein the system additionally comprises saturated silver/silver chloride reference electrode.

3. The electrochemical system according to claims 1 or 2, wherein the electrodes used are conducting electrodes.

4. The electrochemical system according to any of claims 1-3, wherein the electrodes are electrically connected using a physiological buffer reaction electrolyte.

5. The electrochemical system according to any of claims 1-4, wherein dielectric material containment cell for the test environment comprises a physiological buffer reaction electrolyte employed for the electron transfer and pH maintaining.

6. The electrochemical system according to any of claims 1-5, wherein measurement system is a low-volume measurement system, designed to hold a maximum volume of 200 microliters.

7. A method for the determination of electrochemical signal, wherein the method comprises:

   (1) determining of analytic signal of cyclic voltammogram in the presence of aliquot;
   (2) calculation of the second order derivative of the processed cyclic voltammetry data;

(3) determination of stable measurement baseline;

(4) determination of the signal position, where the variation of second order derivative is the most significant; applying the second derivative;

(5) evaluation of analytic signal of the cyclic voltammograms.

8. The method for the determination of electrochemical signal according to claim 7, wherein the determination of electrochemical signal is performed with two-, three-, multi-electrode systems comprising at least one electrode modified by materials.

9. The method for the determination of electrochemical signal according to any of claims 7 or 88, wherein cyclic voltammogram signals are evaluated in such a way that they reflect the interaction of immobilized SARS-CoV-2 virus rS proteins with specific antibodies against these proteins, which are present in aliquot.

10. The method for the determination of electrochemical signal according to any of claims 7-9, wherein the determining of analytical signal obtained from cyclic voltametric data is based on evaluating a mathematical model applying a second order derivative to the polarization with the redox response of the analyte in the cyclic voltammogram, and differentiating it.

11. The method for the determination of electrochemical signal according to claim 7, wherein the method additionally comprises mathematical model of smoothing of the unprocessed data which is performed after step (2).

12. The method for the determination of electrochemical signal according to any of claims 7 - 11, wherein the baseline of the signal is determined using the second derivative of the cyclic voltammogram function.

13. The method for the determination of electrochemical signal according to any of claims 7 - 12, wherein the determining of the analytical signal is based on evaluating mathematical pathway model relating the difference between the recorded current density at the second derivative minimum and the interpolated baseline current density.

14. The electrochemical method according to any claims 7-13, wherein the method is applied to any number of analytes and to any type of electrochemical cells.

15. The electrochemical method according to any claims 7-14, wherein the method to determination of concentrations of analytes, such as protein organic compounds and ions in biological fluids and in other water-based solutions.

Fig. 1

Fig. 2

Fig. 3

Fig. 4

Fig. 5

Fig. 6

Fig. 7

Europäisches
Patentamt

European
Patent Office

Office européen
des brevets

# EUROPEAN SEARCH REPORT

**Application Number**

EP 23 18 9781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZUKAUSKAS SARUNAS ET AL: "Electrochemical Biosensor for the Determination of Specific Antibodies against SARS-CoV-2 Spike Protein", INTERNATIONAL JOURNAL OF MOLECULAR SCIENCES, vol. 24, no. 1, 31 December 2022 (2022-12-31), page 718, XP093116450, Basel, CH ISSN: 1422-0067, DOI: 10.3390/ijms24010718 * Sections 3.3.-3.6.,2.2.; figures 4-5 * | 1-15 | INV. G01N27/327 G01N33/543 |
| X | US 2022/373510 A1 (KARUMANCHI DEVI KALYAN [US] ET AL) 24 November 2022 (2022-11-24) * paragraphs [0020], [0086]; figures 1-4,13-15 * | 1-6 | |
| X | US 2023/120068 A1 (NUNEZ-BAJO ESTEFANIA [GB] ET AL) 20 April 2023 (2023-04-20) * paragraphs [0052], [0059], [0066], [0107]; figures 1-4 * | 1-6 | **TECHNICAL FIELDS SEARCHED (IPC)** G01N |
| X | ESPINOZA ELI M. ET AL: "Practical Aspects of Cyclic Voltammetry: How to Estimate Reduction Potentials When Irreversibility Prevails", JOURNAL OF THE ELECTROCHEMICAL SOCIETY, vol. 166, no. 5, 1 January 2019 (2019-01-01), pages H3175-H3187, XP093117149, ISSN: 0013-4651, DOI: 10.1149/2.0241905jes * Section Methods * | 7,8,10, 11,13 | |

The present search report has been drawn up for all claims

| Place of search | Date of completion of the search | Examiner |
|---|---|---|
| Munich | 10 January 2024 | Lazar, Zala |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                          

& : member of the same patent family, corresponding document

EPO FORM 1503 03.82 (P04C01)

Europäisches
Patentamt

European
Patent Office

Office européen
des brevets

**Application Number**

**EP 23 18 9781**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

**Application Number**

**EP 23 18 9781**

The Search Division considers that the present European patent application does not comply with the requirements of unity of invention and relates to several inventions or groups of inventions, namely:

1. claims: 1-15

   Electrochemical cell comprising gold working electrode and an additional platinum electrode

1.1. claims: 7-15

   Method for determining an electrochemical signal via cyclic voltammetry

   ---

Please note that all inventions mentioned under item 1, although not necessarily linked by a common inventive concept, could be searched without effort justifying an additional fee.

## ANNEX TO THE EUROPEAN SEARCH REPORT
## ON EUROPEAN PATENT APPLICATION NO.

**EP 23 18 9781**

This annex lists the patent family members relating to the patent documents cited in the above-mentioned European search report.
The members are as contained in the European Patent Office EDP file on
The European Patent Office is in no way liable for these particulars which are merely given for the purpose of information.

**10-01-2024**

| Patent document cited in search report | | Publication date | Patent family member(s) | | Publication date |
|---|---|---|---|---|---|
| US 2022373510 | A1 | 24-11-2022 | US | 11513097 B1 | 29-11-2022 |
| | | | US | 11525799 B1 | 13-12-2022 |
| US 2023120068 | A1 | 20-04-2023 | US | 2023120068 A1 | 20-04-2023 |
| | | | WO | 2021180921 A1 | 16-09-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9841403 B2
- CN 102141538 B

- CN 109444245 B